Europäisches Patentamt

European Patent Office

Office européen des brevets

(19)

(11) Veröffentlichungsnummer: **0 561 825 B1**

(12) **EUROPÄISCHE PATENTSCHRIFT**

(45) Veröffentlichungstag der Patentschrift: **27.09.95**

(51) Int. Cl.6: **C07C 305/10**, C07C 303/24

(21) Anmeldenummer: **91920523.7**

(22) Anmeldetag: **25.11.91**

(86) Internationale Anmeldenummer:
**PCT/EP91/02208**

(87) Internationale Veröffentlichungsnummer:
**WO 92/09569 (11.06.92 92/13)**

(54) **VERFAHREN ZUR KONTINUIERLICHEN HERSTELLUNG VON PARTIALGLYCERIDSULFATEN.**

(30) Priorität: **03.12.90 DE 4038477**

(43) Veröffentlichungstag der Anmeldung:
**29.09.93 Patentblatt 93/39**

(45) Bekanntmachung des Hinweises auf die
Patenterteilung:
**27.09.95 Patentblatt 95/39**

(84) Benannte Vertragsstaaten:
**DE ES FR**

(56) Entgegenhaltungen:
**EP-A- 0 267 518
US-A- 2 285 773**

(73) Patentinhaber: **Henkel Kommanditgesellschaft auf Aktien**

**D-40191 Düsseldorf (DE)**

(72) Erfinder: **BEHLER, Ansgar**
**Siegfriedstrasse 80**
**D-4250 Bottrop (DE)**
Erfinder: **PLOOG, Uwe**
**Haydnweg 6**
**D-5657 Haan (DE)**
Erfinder: **FABRY, Bernd**
**Danziger Strasse 31**
**D-4052 Korschenbroich (DE)**
Erfinder: **CLASEN, Frank**
**Am Bandsbusch 46**
**D-4010 Hilden (DE)**

**Beschreibung**

Die Erfindung betrifft ein Verfahren zur kontinuierlichen Herstellung von Partialglyceridsulfaten durch Umsetzung von Partialglyceriden mit gasförmigem Schwefeltrioxid und anschließende Neutralisation der Reaktionsprodukte mit wäßrigen Basen.

Sulfatierte Partialglyceride, insbesondere Monoglyceridsulfate, stellen Aniontenside dar, die sich durch hohes Schaumvermögen, gute Reinigungsleistung und hervorragende dermatologische Verträglichkeit auszeichnen [**Anionic Surfactants, Pt.I, Surfactant science series Vol.7, W.M. Linfield (Ed.), Marcel Dekker Inc., New York, 1976, S. 219**].

Zur Herstellung von Monoglyceridsulfaten geht man gewöhnlich von Glycerin aus, das man zunächst mit Oleum [**US 2,693,479**] oder Chlorsulfonsäure [**JP 78/77014**] zum Glycerinsulfat umsetzt und dann unter Zusatz eines Triglycerids zum Monoglyceridsulfat umestert [**Lipidos 26, 19 (1966)**]. Die deutsche Patentanmeldung **DE-A-38 21 446** offenbart ferner ein Verfahren zur Herstellung von Monoglyceridsulfaten durch Umsetzung von Glycerin mit Chlorsulfonsäure in einem organischem Lösemittel, bei dem zur Umesterung Fettsäuren oder Fettsäureester eingesetzt werden. Die Sulfierung mit Oleum oder Chlorsulfonsäure führt jedoch zu Produkten mit hoher Elektrolytbelastung und ist daher unvorteilhaft.

Aus **Philipp.J.Sci. 311 (1965)** ist bekannt, daß sich Gemische aus Triglyceriden und Glycerin in Gegenwart alkalischer Katalysatoren umestern lassen. In dem genannten Dokument wird ferner vorgeschlagen, die erhaltenen Glycerinfettsäurepartialester mit Schwefelsäure oder Oleum zu sulfatieren.

Die Sulfatierung von Glycerin mit Schwefeltrioxid und die anschließende Umesterung des gebildeten Glycerinsulfats mit Triglyceriden ist aus **US 2,979,521** bekannt. Auf diesen Wegen werden jedoch nur Produkte mit unbefriedigenden Sulfiergraden erhalten.

Die direkte Umsetzung von Monoglyceriden mit Schwefeltrioxid in flüssigem Schwefeldioxid [**US 3,634,287**] oder mit Oleum als Sulfieragens [**JP 50/70322**] ist ebenfalls bekannt. Die europäische Patentanmeldung **EP-A-0 267 518** offenbart ferner die Umsetzung von Partialglyceriden mit Schwefeltrioxid in stickstoffhaltigen Lösemitteln. Die Verwendung von Lösemitteln ist für die technische Herstellung von derartigen Partialglyceridsulfaten ebenfalls von Nachteil, da sie nicht im Produkt verbleiben können, sondern nach der Reaktion mit einem hohem Aufwand an Zeit und Energie abgetrennt werden müssen.

Die Aufgabe der Erfindung bestand somit darin, ein Verfahren zur Herstellung von Partialglyceridsulfaten zu entwickeln, das frei von den geschilderten Nachteilen ist.

Gegenstand der Erfindung ist ein Verfahren zur kontinuierlichen Herstellung von Partialglyceridsulfaten, das sich dadurch auszeichnet, daß man technische Partialglyceride kontinuierlich mit gasförmigem Schwefeltrioxid umsetzt, die sauren Sulfierprodukte einer Alterung unterwirft und anschließend mit wäßrigen Basen in Gegenwart eines Puffers neutralisiert.

Die Erfindung beruht auf der Erkenntnis, daß durch Alterung der rohen Sulfierungsprodukte und Einsatz eines Puffers in der Neutralisation Partialglyceridsulfate mit besonders hohen Sulfiergraden erhalten werden.

Partialglyceride stellen bekannte chemische Stoffe dar, die nach den einschlägigen Methoden der organischen präparativen Chemie, beispielsweise durch Umesterung von Triglyceriden mit Glycerin, erhältlich sind. Zur Herstellung der Partialglyceridsulfate eignen sich Mono- und Difettsäureglycerinester sowie deren technische Gemische auf Basis von aliphatischen Carbonsäuren mit 6 bis 22 Kohlenstoffatomen und 0, 1, 2 oder 3 Doppelbindungen. Im einzelnen kommen die Mono- und Diglyceride der Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, Arachinsäure, Gadoleinsäure, Behensäure oder Erucasäure in Betracht. Bevorzugt ist der Einsatz von Laurinsäuremono- und/oder -diglycerid.

Wie in der Fettchemie üblich, können die Fettsäurekomponenten der Partialglyceride ihrerseits ebenfalls technische Gemische darstellen, wie sie beispielsweise bei der Druckspaltung von natürlichen Fetten und Ölen, wie beispielsweise Kokosöl, Palmöl, Palmkernöl, Rüböl, Sonnenblumenöl, Korianderöl oder Rindertalg anfallen. Bevorzugt ist der Einsatz von gehärtetem Kokosfettsäuremono- und/oder -diglycerid. Geringe Mengen an Triglyceriden oder freiem Glycerin, die als Verunreinigungen in den technischen Partialglyceridgemischen enthalten sein können, stören die Sulfatierung nicht.

Die Sulfierung der Fettsäureglycerinester mit gasförmigem Schwefeltrioxid kann in der für Fettsäureniedrigalkylester bekannten Weise [**J.Falbe (ed.), "Surfactants in consumer products", Springer Verlag, Berlin-Heidelberg, 1987, S. 61**] erfolgen, wobei Reaktoren, die nach dem Fallfilmprinzip arbeiten, bevorzugt sind. Dabei wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt.

Die Sulfierung kann mit 0,95 bis 1,8 mol gasförmigem Schwefeltrioxid pro Mol der in den Partialestergemischen enthaltenen Hydroxylgruppen durchgeführt werden. Im Hinblick auf hohe Sulfiergrade und Hellfarbigkeit der Produkte hat es sich als optimal erwiesen, die Sulfierung mit 1 bis 1,3 mol Schwefeltrioxid pro Mol Hydroxylgruppen im Partialglyceridester durchzuführen.

Die Sulfierung kann bei Temperaturen von 70 bis 98°C durchgeführt werden. Im Hinblick auf die Herstellung von Produkten mit hohem Sulfiergrad hat es sich als optimal erwiesen, eine Temperatur von 90 bis 95°C zu wählen.

Im Anschluß an die Sulfierung wird das rohe Sulfierprodukt einer Alterung unterworfen. Dieser Schritt kann kontinuierlich, beispielsweise in einer Rohrschlange, oder diskontinuierlich, beispielsweise in einem Kessel durchgeführt werden. Die Alterung kann über einen Zeitraum von 1 bis 240 min, vorzugsweise 5 bis 30 min und bei Temperaturen von 70 bis 98, vorzugsweise 90 bis 95°C durchgeführt werden. Erfolgt die Alterung innerhalb der angegebenen Grenzen bei niedrigen Temperaturen, sind zur Erzielung hoher Sulfiergrade lange Verweilzeiten erforderlich und umgekehrt.

Die bei der Sulfierung anfallenden sauren Sulfierprodukte werden nach der Alterung gemeinsam mit wäßrigen Basen in eine wäßrige Pufferlösung eingerührt und neutralisiert, wobei ein pH-Wert von 5,5 bis 9, vorzugsweise 6,5 bis 8 eingehalten werden muß, da andernfalls eine Hydrolyse der Esterbindung oder eine Abspaltung der Sulfatgruppe stattfindet. Als Puffer kommen beispielsweise eine 1 bis 5 gew.-%ige wäßrige Lösungen von Natriumtriphosphat, Natriumhydrogencarbonat oder Citronensäure in Betracht.

Als Basen für die Neutralisation kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-$C_{2-4}$-Alkanolamine, beispielsweise Mono-, Di- und Triethanolamin sowie primäre, sekundäre oder tertiäre $C_{1-4}$-Alkylamine in Betracht. Die Neutralisationsbasen gelangen dabei vorzugsweise in Form 5 bis 55 gew.-%iger wäßriger Lösungen zum Einsatz, wobei 5 bis 25 gew.-%ige wäßrige Natriumhydroxidlösungen bevorzugt sind.

Die Sulfierprodukte stellen komplexe Gemische dar, die zu mehr als 50 Gew.-% Sulfatierungsprodukte der primären sowie sekundären Hydroxylgruppen der Partialglyceride, also Mono- und Diglyceridsulfate, enthalten. Darüberhinaus können in der Reaktionsmischung offenkettige und cyclische Glycerinsulfate sowie alpha-Glycerinestersulfonate, Produkte mit Sulfat- und Sulfonatgruppen, Seifen, sulfierte Seifen und Glycerin enthalten sein. Geht man von ungesättigten Partialglyceriden aus, findet untergeordnet auch noch eine Addition des Schwefeltrioxids an die Doppelbindung der Fettsäurekomponente unter Bildung innenständiger Glycerinestersulfonate statt.

Die Sulfierprodukte können nach Neutralisation in an sich bekannter Weise durch Zusatz von Wasserstoffperoxid- oder Natriumhypochloritlösung gebleicht werden. Dabei werden, bezogen auf den Feststoffgehalt in der Lösung der Sulfierprodukte, 0.2 bis 2 Gew.-% Wasserstoffperoxid, berechnet als 100 %ige Substanz, oder entsprechende Mengen Natriumhypochlorit eingesetzt. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

Die Partialglyceridsulfate zeigen oberflächenaktive Eigenschaften und eignen sich zur Herstellung von pulverförmigen oder flüssigen Wasch- und Reinigungsmitteln sowie von Produkten zur Körper- und Haarpflege.

Die folgenden Beispiele sollen den Gegenstand der Erfindung näher erläutern, ohne ihn darauf einzuschränken.

## Beispiele

Ausgangsstoffe:

a) Laurinsäuremonoglycerid
Monomuls[R] 90-L-16, Chemische Fabrik Grünau GmbH, Illertissen

| Zusammensetzung | 68 Gew.-% $C_{12}$-Monoglycerid 32 Gew.-% $C_{12}$-Diglycerid |
|---|---|
| Mittleres Molgewicht Hydroxylzahl | 136 413 |

b) Kokosfettsäuremonoglycerid

| Zusammensetzung | 46 Gew.-% $C_{12/14}$-Monoglycerid |
| | 32 Gew.-% $C_{12/14}$-Diglycerid |
| | 7 Gew.-% $C_{12/14}$-Triglycerid |
| | 10 Gew.-% Glycerin |
| Mittleres Molgewicht | 140 |
| Hydroxylzahl | 401 |

Beispiel 1:

**Sulfierung von Laurinsäuremonoglycerid**. In einem kontinuierlich arbeitenden Fallfilmreaktor (Länge 120 cm, Querschnitt 1 cm, Eduktdurchsatz 600 g/h) mit Mantelkühlung und seitlicher $SO_3$-Begasung wurden 1650 g (5 mol) des technischen Monoglycerids A bei 95°C mit Schwefeltrioxid umgesetzt. Das Einsatzverhältnis betrug 1,3 mol $SO_3$ pro Mol im Partialester enthaltener Hydroxylgruppen. Das Schwefeltrioxid wurde durch Erhitzen aus einer entsprechenden Menge 65 gew.-%igen Oleums ausgetrieben, mit Stickstoff auf eine Konzentration von 5 Vol.-% verdünnt und über eine Düse mit dem Monoglyceridfilm in Kontakt gebracht. Das rohe Sulfierprodukt wurde einer Alterung unterworfen und hierzu 30 min bei 90°C gelagert. Anschließend wurde es zusammen mit 37 gew.-%iger Natriumhydroxidlösung in eine 1 gew.-%igen Lösung von Natriumtriphosphat eingerührt und bei pH = 6,5 bis 8 neutralisiert. Die Kenndaten des Reaktionsproduktes in Tab.1 zusammengefaßt.

Vergleichsbeispiel 1:

Beispiel 1 wurde wiederholt, jedoch auf die Alterung nach der Sulfierung und vor der Neutralisation verzichtet. Die Kenndaten des Reaktionsproduktes in Tab.1 zusammengefaßt.

Beispiel 2:

Analog Beispiel 1 wurden 1980 g (5 mol) eines technischen Kokosfettsäuremonoglycerids B Schwefeltrioxid umgesetzt. Das Einsatzverhältnis betrug wiederum 1,3 mol Schwefeltrioxid pro Mol im Partialester enthaltener Hydroxylgruppen. Nach Alterung und Neutralisation wurde ein Produkt erhalten, dessen Kenndaten in Tab.1 zusammengefaßt sind.

Vergleichsbeispiel 2:

Beispiel 2 wurde wiederholt, jedoch auf die Alterung nach der Sulfierung und vor der Neutralisation verzichtet. Die Kenndaten des Reaktionsproduktes sind in Tab.1 zusammengefaßt.

Der Aniontensidgehalt (WAS) und die unsulfierten Anteile (US) - hier die Summe aus freiem Partialglycerid und Seife - wurden nach den DGF-Einheitsmethoden, Stuttgart, 1950-1984, H-III-10 bzw. G-II-6b ermittelt. Der Sulfatgehalt wurde als Natriumsulfat berechnet, die Bestimmung des Seifengehaltes erfolgte über eine Dünnschichtchromatographie, die Bestimmung des Wassergehaltes nach der Fischer-Methode.

Der Sulfiergrad S° wurde nach folgenden Formeln berechnet:

$$x = \frac{M^{Ed} \; WAS}{M^{Pr}} \quad \text{und} \quad S° = \frac{x \cdot 100}{x + US}$$

Hierbei stehen $M^{Ed}$ und $M^{Pr}$ für die Molgewichte der Ausgangsstoffe beziehungsweise der sulfierten Produkte.

EP 0 561 825 B1

Tab.1

| Kenndaten der Sulfierprodukte Prozentangaben als Gew.-% | | | | | | |
|---|---|---|---|---|---|---|
| Bsp. | WAS % | PG % | Seife % | $SO_4^{2-}$ % | $H_2O$ % | S° % |
| 1 | 15,1 | 2,5 | 0,3 | 5,5 | 76,6 | 80 |
| 2 | 16,5 | 1,4 | 0,4 | 6,2 | 75,5 | 86 |
| V1 | 13,4 | 3,2 | 0,3 | 6,4 | 76,7 | 75 |
| V2 | 14,0 | 2,7 | 0,3 | 7,2 | 75,7 | 79 |
| Legende: PG = Gehalt an unsulfiertem Partialglycerid | | | | | | |

**Patentansprüche**

1. Verfahren zur kontinuierlichen Herstellung von Partialglyceridsulfaten, **dadurch gekennzeichnet**, daß man technische Partialglyceride kontinuierlich mit gasförmigem Schwefeltrioxid umsetzt, die sauren Sulfierprodukte einer Alterung unterwirft und anschließend mit wäßrigen Basen in Gegenwart eines Puffers neutralisiert.

2. Verfahren nach Anspruch 1, **dadurch gekennzeichnet**, daß man die Sulfierung in einem kontinuierlich arbeitenden Reaktor durchführt, der nach dem Fallfilmprinzip arbeitet.

3. Verfahren nach mindestens einem der Ansprüche 1 und 2, **dadurch gekennzeichnet**, daß man die Sulfierung bei einer Temperatur von 70 bis 98°C durchführt.

4. Verfahren nach mindestens einem der Ansprüche 1 bis 3, **dadurch gekennzeichnet**, daß man die Sulfierung mit einem Einsatzverhältnis von 0,95 bis 1,8 mol Schwefeltrioxid pro Mol im Partialester enthaltener Hydroxylgruppen durchführt.

5. Verfahren nach mindestens einem der Ansprüche 1 bis 4, **dadurch gekennzeichnet**, daß man die Alterung über einen Zeitraum von 1 bis 240 min durchführt.

6. Verfahren nach mindestens einem der Ansprüche 1 bis 5, **dadurch gekennzeichnet**, daß man die Alterung bei einer Temperatur von 70 bis 98°C durchführt.

7. Verfahren nach mindestens einem der Ansprüche 1 bis 6, **dadurch gekennzeichnet**, daß man die Neutralisation mit 5 bis 55 gew.-%igen wäßrigen Basen aus der von Alkalimetallhydroxiden, Erdalkalimetalloxiden und -hydroxiden, Ammoniak, Mono-, Di- und Tri-$C_{2-4}$-Alkanolaminen sowie primären, sekundären und tertiären $C_{1-4}$-Alkylaminen gebildeten Gruppe durchführt.

8. Verfahren nach mindestens einem der Ansprüche 1 bis 7, **dadurch gekennzeichnet**, daß man die Neutralisation unter Einhaltung eines pH-Bereiches von 5,5 bis 9 durchführt.

**Claims**

1. A process for the continuous production of partial glyceride sulfates, **characterized in that** technical partial glycerides are continuously reacted with gaseous sulfur trioxide, the acidic sulfonation products are subjected to ageing and are then neutralized with aqueous bases in the presence of a buffer.

2. A process as claimed in claim 1, **characterized in that** the sulfonation is carried out in a continuously operating falling-film reactor.

3. A process as claimed in at least one of claims 1 and 2, **characterized in that** the sulfonation is carried out at a temperature of 70 to 98°C.

4. A process as claimed in at least one of claims 1 to 3, **characterized in that** the sulfonation is carried out with a ratio of 0.95 to 1.8 mol sulfur trioxide per mol hydroxyl groups in the partial ester.

5

5. A process as claimed in at least one of claims 1 to 4, **characterized in that** the ageing step is carried out over a period of 1 to 240 minutes.

6. A process as claimed in at least one of claims 1 to 5, **characterized in that** the ageing step is carried out at a temperature of 70 to 98°C.

7. A process as claimed in at least one of claims 1 to 6, **characterized in that** the neutralization step is carried out with 5 to 55% by weight aqueous bases from the group consisting of alkali metal hydroxides, alkaline earth metal oxides and hydroxides, ammonia, mono-, di- and tri-$C_{2-4}$-alkanolamines and primary, secondary and tertiary $C_{1-4}$ alkyl amines.

8. A process as claimed in at least one of claims 1 to 7, **characterized in that** the neutralization step is carried out at a pH value in the range from 5.5 to 9.

**Revendications**

1. Procédé de production en continu de sulfates de glycérides partiels, caractérisé en ce que l'on fait réagir des glycérides partiels industriels en continu avec de l'anhydride sulfurique gazeux, en ce que l'on soumet les produits de sulfatation acides à un vieillissement, et en ce qu'ensuite on neutralise avec des bases aqueuses en présence d'un tampon.

2. Procédé selon la revendication 1, caractérisé en ce que l'on effectue la sulfatation dans un réacteur qui fonctionne en continu selon le principe du ruissellement.

3. Procédé selon au moins une des revendications 1 et 2, caractérisé en ce que l'on effectue la sulfaration à une température de 70 à 98°C.

4. Procédé selon au moins une des revendications 1 à 3, caractérisé en ce que l'on effectue la sulfatation avec un rapport d'utilisation de 0,95 à 1,8 mol d'anhydride sulfurique par moi de groupes hydroxyle contenus dans l'ester partiel.

5. Procédé selon au moins une des revendications 1 à 4, caractérisé en ce que l'on effectue le vieillissement en un laps de temps de 1 à 240 minutes.

6. Procédé selon au moins une des revendications 1 à 5, caractérisé en ce que l'on effectue le vieillissement à une température de 70 à 98°C.

7. Procédé selon au moins une des revendications 1 à 6, caractérisé en ce que l'on effectue la neutralisation avec des bases aqueuses de 5 à 55 % en poids choisies dans le groupe formé des hydroxydes de métal alcalin, des oxydes et des hydroxydes de métal alcalinoterreux, de l'ammoniac, des mono- des di-, des tri-alcanol en $C_2$ à $C_4$ amines ainsi que des alcoyles en $C_1$-$C_4$ amines primaires, secondaires et tertiaires.

8. Procédé selon au moins l'une des revendications 1 à 7, caractérisé en ce que l'on effectue la neutralisation tout en maintenant une plage de pH de 5,5 à 9.